(19) Europäisches Patentamt
European Patent Office
Office européen des brevets

(11) **EP 2 749 903 B1**

(12) **FASCICULE DE BREVET EUROPEEN**

(45) Date de publication et mention
de la délivrance du brevet:
**22.01.2020 Bulletin 2020/04**

(21) Numéro de dépôt: **13198458.5**

(22) Date de dépôt: **19.12.2013**

(51) Int Cl.:
***G01T 1/17*** *(2006.01)*

(54) **Circuit de connexion multiplexé et dispositif de détection d'au moins une particule utilisant le circuit de connexion**

Multiplex-Verbindungsschaltkreis und Vorrichtung zur Erkennung mindestens eines Partikels, der diesen Verbindungsschaltkreis nutzt

Multiplexed connection circuit and device for detecting at least one particle using the connection circuit

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **27.12.2012 FR 1262815**

(43) Date de publication de la demande:
**02.07.2014 Bulletin 2014/27**

(73) Titulaire: **COMMISSARIAT À L'ÉNERGIE ATOMIQUE ET AUX ÉNERGIES ALTERNATIVES 75015 Paris (FR)**

(72) Inventeurs:
• **Procureur, Sébastien**
**91120 Palaiseau (FR)**
• **Aune, Stefan**
**91120 Palaiseau (FR)**
• **Dupre, Raphaël**
**91300 Massy (FR)**

(74) Mandataire: **Novaimo**
**ActiTech 8**
**60 avenue Marie Curie**
**Archamps Technopole**
**74166 Saint Julien-en-Genevois Cedex (FR)**

(56) Documents cités:
**DE-B3-102005 055 656 US-A- 3 777 161**

• **LEE ET AL: "multi-strip scintillation counters(mssc)", NUCLEAR INSTRUMENTS AND METHODS, NORTH-HOLLAND PUBLISHING COMPANY. AMSTERDAM, NL, vol. 119, no. 1, 1 janvier 1974 (1974-01-01), pages 29-33, XP002135485, DOI: 10.1016/0029-554X(74)90729-0**
• **CROW M L ET AL: "Shifting scintillator prototype large pixel wavelength-shifting fiber detector for the POWGEN3 powder diffractometer", NUCLEAR INSTRUMENTS & METHODS IN PHYSICS RESEARCH. SECTION A: ACCELERATORS, SPECTROMETERS, DETECTORS, AND ASSOCIATED EQUIPMENT, ELSEVIER BV * NORTH-HOLLAND, NETHERLANDS, vol. 529, no. 1-3, 21 août 2004 (2004-08-21), pages 287-292, XP004527303, ISSN: 0168-9002, DOI: 10.1016/J.NIMA.2004.04.167**

## Description

## Domaine technique de l'invention

L'invention concerne le domaine des circuits de connexion permettant notamment de réaliser un multiplexage.

L'invention a pour objet plus particulièrement un circuit de connexion multiplexé et un dispositif de détection et notamment de localisation d'au moins une particule comprenant ledit circuit de connexion.

## État de la technique

Les détecteurs gazeux à pistes ou micro-pistes sont utilisés dans de nombreux domaines, et plus particulièrement en physique des particules et en imagerie médicale pour localiser des particules. Ils nécessitent l'installation d'un grand nombre de voies de sortie (jusqu'à plusieurs dizaines voire centaines de milliers), aussi appelées voies électroniques ou voies d'électronique dans le domaine. Le coût total de ces voies de sortie est souvent bien supérieur à celui de fabrication des éléments de détection. Afin de limiter les coûts de fabrication de tels détecteurs, il a résulté un besoin de trouver des solutions pour diminuer le nombre de voies de sortie.

Dans l'optique de satisfaire ce besoin, il a été proposé une solution permettant de localiser une particule au niveau d'un dispositif de localisation mettant en œuvre un circuit de connexion avec un multiplexage spécifique.

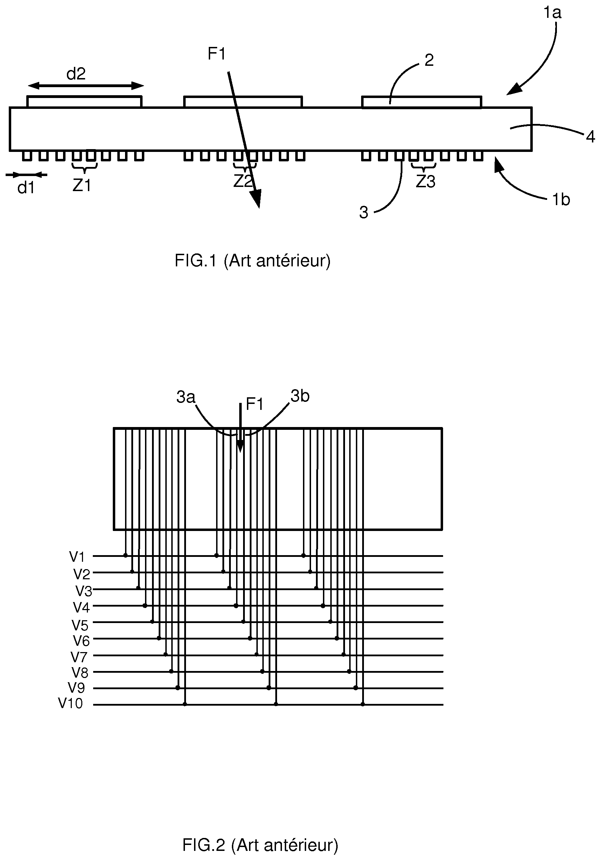

Une telle solution de multiplexage a consisté au développement d'un dispositif de localisation de particule(s) dit « double-face ». Comme illustré à la figure 1, une première face 1a du dispositif de localisation comporte i grosses pistes 2 (i étant égal à 3 dans l'exemple illustré), et une deuxième face 1b du dispositif de localisation comporte p petites pistes 3 (p étant égal à 8 dans l'exemple) qui se répètent i fois pour former des blocs identiques sous les grosses pistes 2. Les deux faces 1a et 1b sont, dans l'exemple, délimitées par un circuit imprimé 4. Le passage F1 d'une particule au travers des deux faces 1a, 1b du dispositif de localisation permet la localisation de ladite particule avec une précision correspondant au pas des petites pistes 3. En fait, les petites pistes 3 étant multiplexées, une grosse piste 2 permet une première localisation dans un ensemble de petites pistes 3, et la localisation au niveau d'une ou plusieurs petites pistes 3 en regard d'une grosse piste 2 associée permet de localiser avec précision la particule.

La différence entre « petite » et « grosse » correspond à ce qu'une petite piste présente une dimension latérale d1 inférieure à la dimension latérale d2 d'une grosse piste.

En fait, sans les grosses pistes 2, le passage de la particule générerait une donnée qui, à cause du multiplexage, serait assimilée comme une localisation dans une zone Z1, une zone Z2 ou une zone Z3 du dispositif de localisation. Le multiplexage illustré à la figure 2 permet de mieux comprendre la problématique, en effet, la particule reçue selon F1 l'est par les deux pistes 3a, 3b entourant le bout de la flèche F1. Cette particule sera donc associée à des voies de sortie V4 et V5 qui sont aussi associées à d'autres pistes de l'ensemble des petites pistes, d'où l'intérêt de réaliser un filtre avec les grosses pistes 2 de la figure 1.

Le type de multiplexage illustré aux figures 1 et 2 permet à l'aide d'un circuit à i+p pistes de réaliser un détecteur équivalent à i*p pistes.

Cependant, une telle solution présente différents inconvénients dont notamment :

- une difficulté de fabrication car il faut en effet fabriquer deux ensembles de détection de part et d'autre du circuit imprimé,

- l'utilisation d'une grande quantité de matière : le circuit imprimé sur lequel sont formées les pistes doit être suffisamment rigide et donc épais. Ceci est souvent un handicap en détection, où la quantité de matière doit être minimisée.

- l'inadaptabilité à des flux élevés de particules : si plusieurs particules traversent le dispositif de localisation simultanément, il n'est plus possible de localiser précisément ces particules, ce qui conduit à des inefficacités de détection,

- la mise en défaut de la localisation si l'angle de la particule incidente est trop élevé : en effet, la particule peut alors laisser un signal sur une grosse piste et sur les petites pistes en regard d'une grosse piste voisine, ce qui conduit à une ambiguïté de localisation, notamment en fonction de l'épaisseur du circuit imprimé 4 entre les deux faces 1a, 1b,

- enfin le degré de multiplexage n'est pas optimal, en effet, si l'on dispose au total de n voies de sortie, on peut équiper le dispositif de localisation jusqu'à $n^2/4$ pistes avec i=p=n/2. Un autre circuit de connexion multiplexé est connu de DE 10 2005 055656 B3.

## Objet de l'invention

Le but de la présente invention est de proposer une solution qui remédie au moins en partie aux inconvénients listés ci-dessus.

On tend vers ce but grâce à un circuit de connexion multiplexé comprenant une pluralité de canaux munie de p canaux, notamment configurés de sorte à détecter au moins une particule, et une pluralité de voies de sortie munie de n voies de sortie, avec p>n, chaque canal étant connecté à une unique voie de sortie, ledit circuit comprenant des groupes de canaux adjacents, chaque canal du circuit de connexion appartenant à au moins un groupe de canaux adjacents et chaque groupe de canaux adjacents étant défini par un ensemble formé par les connexions des canaux du groupe de canaux adjacents aux voies de sortie correspondantes, cet ensemble formant un groupe de voies de sortie associé au groupe de canaux adjacents, et chaque groupe de canaux adjacents est associé à un groupe de voies de sortie unique, différent de celui des autres groupes de canaux adjacents.

De préférence, le circuit comporte des points de connexion entre les canaux et les voies de sortie permettant de réaliser la connexion de chaque canal de la pluralité de canaux à une unique voie de sortie de la pluralité de voies de sortie, et les points de connexion :

- définissent les groupes de canaux adjacents,
- forment les groupes de voies de sortie, lesdites voies de sortie de chacun des groupes de voies de sortie étant choisies parmi les n voies de sortie de la pluralité de voies de sortie sans que l'ordre de la voie de sortie dans le groupe de voies de sortie n'ait d'importance et de sorte que les groupes de voies de sortie sont tous différents les uns des autres,

un groupe de voies de sortie et un groupe de canaux associé comprenant respectivement un nombre de voies de sortie et un nombre de canaux égaux.

Avantageusement, au moins une des voies de sortie de la pluralité de voies de sortie est connectée à au moins deux canaux de la pluralité de canaux de deux groupes de canaux distincts.

De préférence, chaque groupe de canaux comprend k canaux et chaque groupe de voies de sortie comporte k voies de sortie avec $k \geq 2$. Par exemple, k étant égal à 2, les connexions sont réalisées de sorte que la pluralité de canaux comporte indifféremment entre 2 et

$$\frac{n*(n-1)}{2}+1 \quad \text{canaux.}$$

Avantageusement, le circuit comporte z groupes de voies de sortie différents non ordonnés avec z un nombre entier quelconque choisi indifféremment entre 2 et $C_n^k$. Par exemple p=z+k-1.

Selon une réalisation, deux canaux appartenant à deux groupes de canaux différents et reliés à une même voie de sortie sont séparés d'au moins x canaux distincts, notamment avec x supérieur au nombre de canaux de chaque groupe de canaux.

De préférence, la pluralité de canaux est agencée de telle sorte que chacun des canaux de la pluralité de canaux est adjacent à au moins un, et de préférence sensiblement parallèle, autre canal de la pluralité de canaux.

Si les canaux sont des pistes, chacune des pistes est au maximum adjacente à deux autres pistes.

L'invention est aussi relative à un dispositif de détection d'au moins une particule comprenant au moins un circuit de connexion tel que décrit dont chacun des canaux de la pluralité de canaux forme un canal de détection de particule, et ce dispositif comprend :

- un élément de détection destiné à recevoir ladite au moins une particule à localiser et comportant la pluralité de canaux de détection de sorte qu'une particule est détectée par la propagation d'un signal de détection dans au moins deux canaux de détection adjacents et,

- un composant d'analyse relié à chacune des voies de sortie de la pluralité de voies de sortie.

De préférence, le dispositif est configuré de sorte que ladite particule à localiser est détectée par au moins tous les canaux d'un des groupes de canaux de détection adjacents, et que la particule détectée est localisée, par le composant d'analyse, dans une unique zone de l'élément de détection uniquement à partir de données de détection issues des canaux de détection ayant détecté ladite particule et transmises audit composant d'analyse par les voies de sortie du groupe de voies de sortie associé au groupe de canaux ayant détecté ladite particule.

L'invention est aussi relative à un procédé de localisation d'au moins une particule à partir d'un dispositif de détection tel que décrit, le procédé comportant :

- une étape dans laquelle ladite au moins une particule est reçue par l'élément de détection,

- une étape de détection par au moins tous les canaux de détection d'un même groupe de canaux adjacents de ladite au moins une particule,

- une étape de génération de données de détection par chacun des canaux de détection du groupe de canaux de détection adjacents ayant détecté ladite au moins une particule,

- une étape de réception par le composant d'analyse desdites données de détection transmises par les voies de sortie connectées audit groupe de canaux de détection adjacents ayant détecté ladite au moins une particule,

- une étape de détermination, à partir desdites données de détection reçues, d'un groupe de voies de sortie associé,

- une étape de détermination d'une zone de l'élément de détection comprenant le groupe de canaux de détection adjacents connecté au groupe de voies de sortie déterminé, ladite zone de l'élément de détection fournissant une donnée de localisation de ladite au moins une particule.

L'invention est aussi relative à un support d'enregistrement de données lisible par un calculateur, sur lequel est enregistré un programme informatique comprenant des moyens de codes de programme informatique de mise en œuvre des étapes d'un procédé de localisation tel que décrit.

L'invention est aussi relative à un programme informatique comprenant un moyen de codes de programme informatique adapté à la réalisation des étapes d'un procédé de localisation tel que décrit, lorsque le programme est exécuté par un calculateur.

## Description sommaire des dessins

D'autres avantages et caractéristiques ressortiront plus clairement de la description qui va suivre de modes particuliers de réalisation de l'invention donnés à titre d'exemples non limitatifs et représentés sur les dessins annexés, dans lesquels :

-   la figure 1 est une vue de côté d'un dispositif de localisation selon l'art antérieur,
-   la figure 2 est une vue de dessous du dispositif de localisation de la figure 1,
-   la figure 3 illustre une vue d'un dispositif de détection selon un mode d'exécution de l'invention,
-   la figure 4 illustre une vue de côté du dispositif de détection de la figure 3.

## Description de modes préférentiels de l'invention

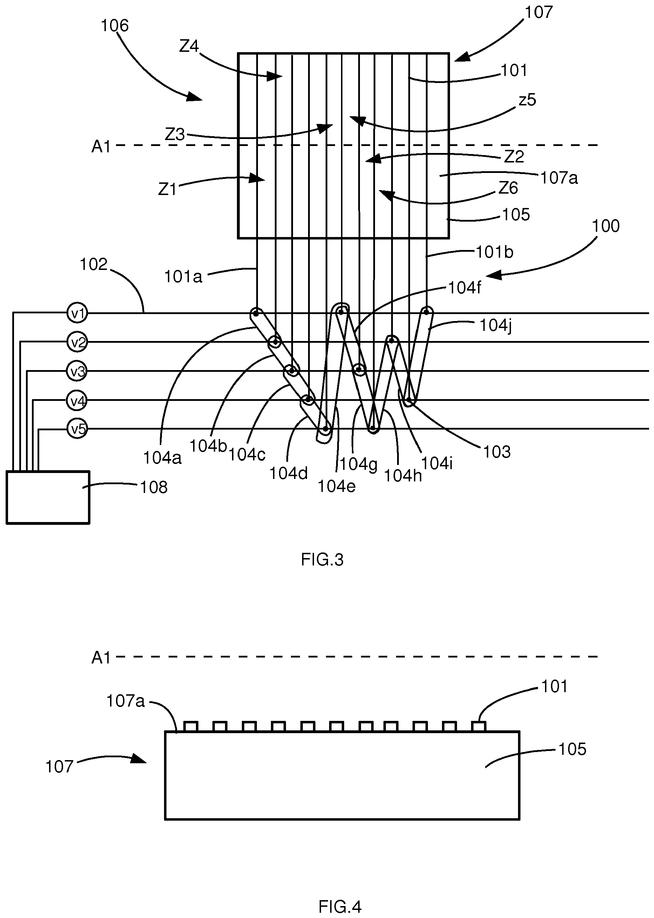

Sur la figure 3, le circuit de connexion 100 multiplexé, notamment pour dispositif de détection d'au moins une particule (permettant notamment de localiser ladite au moins une particule), comprend une pluralité de canaux munie de p canaux 101, notamment configurés de sorte à détecter au moins une particule, et une pluralité de voies de sortie munie de n voies de sortie 102, avec p>n.

Chaque canal est connecté à une unique voie de sortie 102. Le circuit 100 comprend des groupes de canaux adjacents et chaque canal 101 du circuit de connexion 100 appartient à au moins un groupe de canaux adjacents. Chaque groupe de canaux adjacents est défini par un ensemble formé par les connexions des canaux 101 du groupe de canaux adjacents aux voies de sortie 102 correspondantes. Cet ensemble forme un groupe de voies de sortie associé au groupe de canaux adjacents et chaque groupe de canaux adjacents est associé à un groupe de voies de sortie unique, différent de celui des autres groupes de canaux adjacents.

Ce type de multiplexage permet notamment de multiplexer plus de canaux pour un même nombre de voies comparé à l'art antérieur. Ce multiplexage est particulièrement adapté à des dispositifs de détection où le passage d'une particule génère un signal propagé dans au moins deux canaux adjacents. C'est à partir de cette propagation que l'on considère qu'il y a eu détection et c'est donc pour cela que les canaux peuvent être configurés de sorte à détecter au moins une particule.

Le circuit de connexion comprend en outre des points de connexion 103 (représentés par des points noirs à la figure 3 au niveau de chaque jonction d'un canal connecté à une voie de sortie) entre les canaux 101 et les voies de sortie 102 permettant de réaliser la connexion de chaque canal de la pluralité de canaux à une unique voie de sortie 102 de la pluralité de voies de sortie.

Dans la présente description, une voie de sortie (ou voie électronique, ou encore voie d'électronique) est configurée de sorte à faire transiter une donnée, par exemple un signal électrique ou autre. Pour généraliser, les points de connexion 103 sont configurés de sorte à faire passer, le cas échéant, une donnée d'un canal vers une voie au niveau du point de connexion 103 entre ledit canal et ladite voie, cette donnée pouvant être altérée ou modifiée en type (électrique, optique, etc.) lors du passage. Les points de connexion peuvent être de type électrique, les canaux et les voies sont alors électriquement conducteurs par exemple sous forme de piste ou de pixel, ou encore de type optique, les canaux et les voies étant alors configurés de sorte à former des guides d'onde, ou encore de type mécanique : les canaux et les voies étant alors des transmissions mécaniques.

Par ailleurs, selon une mise en œuvre, les points de connexion 103 sont tels qu'ils définissent les groupes de canaux adjacents, notamment lesdits canaux 101 adjacents de chacun des groupes de canaux sont choisis parmi la pluralité de canaux, et qu'ils forment les groupes de voies de sortie, lesdites voies de sortie 102 de chacun des groupes de voies de sortie étant préférentiellement choisies parmi les n voies de sortie 102 de la pluralité de voies de sortie sans que l'ordre de la voie dans le groupe de voie de sortie n'ait d'importance et de sorte que les groupes de voies de sortie sont tous différents les uns des autres. On comprend qu'un groupe de voies de sortie et un groupe de canaux associés comprennent respectivement un nombre de voies de sortie et un nombre de canaux égaux. Autrement dit, les points de connexion 103 peuvent être tels qu'ils associent chaque groupe de voies de sortie à un seul des groupes de canaux adjacents et inversement, chaque groupe de voies de sortie comprenant un nombre de voies égal au nombre de canaux du groupe de canaux associé.

Par exemple, dans un groupe de canaux adjacents, les canaux constituant ce groupe sont en fait adjacents à deux ou à plusieurs autres canaux de ce groupe.

De préférence, toutes les voies de sortie de la pluralité de voies de sortie appartiennent à au moins un groupe de voies de sortie.

Comme évoqué précédemment, tous les canaux de la pluralité de canaux (c'est-à-dire du circuit de connexion) appartiennent à au moins un groupe de canaux. Dans le cas où le nombre de canaux et le nombre de voies de sortie dans leurs groupes respectifs est égal à deux, tous les canaux, à l'exception éventuellement de deux canaux d'extrémité, de la pluralité de canaux peuvent être associés chacun à deux groupes de voies de sortie.

Autrement dit, les groupes de voies de sortie sont tous différents, mais certains groupes de voies de sortie peuvent avoir au moins une voie de sortie en commun. Si les groupes de voies de sortie ont chacun le même nombre de voies de sortie, le nombre maximum de groupes de voies de sortie est défini mathématiquement par les combinaisons de k parmi n, avec k le nombre de voies de sortie par groupe, soit $C_n^k = \dfrac{n!}{k!(n-k)!}$.

Dans l'exemple particulier de la figure 3, chaque groupe de voies de sortie est en fait un couple. En partant sur la base de cinq voies V1, V2, V3, V4, V5 disponibles on sait que l'on peut avoir au plus dix groupes distincts 104a, 104b, 104c, 104d, 104e, 104f, 104g, 104h, 104i, 104j, formés respectivement par (V1 ; V2), (V2 ; V3), (V3 ; V4), (V4 ; V5) (V5 ; V1), (V1 ; V3), (V3 ; V5), (V5 ; V2), (V2 ; V4) et (V4 ; V1). Ces groupes de voies de sortie permettent de multiplexer 11 canaux en formant 10 couples de canaux adjacents. Ainsi, le circuit de connexion peut comporter p-1 couples de canaux adjacents et $C_n^2$ couples de voies de sortie différents tels que définis précédemment, et chaque couple de canaux adjacents étant associé à un couple distinct de voies de sortie et inversement. A part pour les deux groupes de canaux comprenant les canaux d'extrémité 101a et 101b, les deux canaux de chaque groupe de canaux appartiennent chacun à un autre groupe de canaux.

Selon une variante, chaque canal appartient à un unique groupe de canaux adjacents.

De préférence, afin d'améliorer le multiplexage et de diminuer le nombre de voies de sortie nécessaires, chacune (ou au moins une) des voies de sortie 102 de la pluralité de voies de sortie est connectée à au moins deux canaux 101 de la pluralité de canaux de deux groupes de canaux distincts.

Selon un mode d'exécution, chaque groupe de canaux comprend k canaux et chaque groupe de voies de sortie comporte k voies de sortie avec k≥2. Autrement dit, le nombre de voies de sortie de chaque groupe de voies de sortie est égal au nombre de canaux de chaque groupe de canaux.

En fait, si k est égal à 2, les connexions 103 sont réalisées de sorte que la pluralité de canaux comporte indifféremment entre 2 et $\dfrac{n*(n-1)}{2}+1$ canaux (autrement dit, $2 \leq p \leq \dfrac{n*(n-1)}{2}+1$). Il est donc possible de former à partir d'un tel circuit de connexion un dispositif de détection de particule(s) équivalent comportant jusqu'à $\dfrac{n*(n-1)}{2}+1$ canaux en utilisant une construction comme celle indiquée ci-dessus.

Autrement dit, pour n voies numérotées de V1 à Vn, on peut par exemple faire des connexions avec la 1ère voie de sortie V1 disponible et dans ce cas :

- On commence par connecter dans l'ordre individuellement les n premiers canaux aux n voies de sortie V1 à Vn (Ceci n'étant qu'une convention de numérotation),

- Puis le n+1ème canal est à nouveau connecté à la 1ère voie de sortie V1, le n+2ème canal ne peut pas être connecté à la 2ème voie de sortie, car alors le groupe de voies V1-V2 serait présent deux fois. En revanche, il est possible de connecter ce n+2ème canal à la 3ème voie de sortie, puisque le doublet V1-V3 n'est pas encore utilisé.

- On continue la procédure à l'identique : si le canal i du détecteur est connecté à la voie j de sortie, on connecte le canal i+1 à la première voie de sortie h disponible après la voie j. Disponible signifie que le doublet j-h (ou h-j puisque les groupes de voies ne sont pas ordonnés) n'apparait pas encore dans le circuit de connexion.

Cette méthode permet de construire (n-1)/2 séries des n voies de sortie. On peut éventuellement imposer que chaque série contient exactement une fois chaque voie d'électronique. Dans un exemple particulier où n vaut 15, la liste suivante indique à quelles voies sont connectés les canaux successifs :

V1-V2-V3-V4-V5-V6-V7-V8-V9-V10-V11-V12-V13-V14-V15 (1ère série de n canaux)

V1-V3-V5-V7-V9-V11-V13-V15-V2-V4-V6-V8-V10-V12-V14 (2ème série de n canaux)

V1-V4-V7-V10-V13-V2-V5-V8-V11-V14-V3-V6-V9-V12-V15 (3ème série de n canaux)

V3-V7-V11-V15-V4-V8-V12-V1-V5-V9-V13-V6-V10-V14-V2 (4ème série de n canaux)

V6-V11-V1-V7-V12-V2-V8-V13-V3-V9-V14-V4-V10-V15-V5 (5ème série de n canaux)

V10-V1-V6-V12-V3-V8-V14-V5-V11-V2-V7-V13-V4-V9-V15 (6ème série de n canaux)

V6-V14-V7-V15-V8-V1-V9-V2-V10-V3-V11-V4-V12-V5-V13 (7ème série de n canaux)

Dans ce cas particulier, 15 voies permettent de connecter 105 canaux. On voit bien selon cet exemple que chaque doublet i-j apparait une seule fois dans ladite liste.

Selon une autre méthode, dans la r$^{ème}$ série de canaux, on connecte le premier canal à la 1$^{ère}$ voie d'électronique disponible, puis on va de r en r, modulo n. Cette méthode permet de construire très facilement tous les doublets si n est un nombre premier. Cette méthode est illustrée ci-dessous avec n=11 et donne la liste :

V1-V2-V3-V4-V5-V6-V7-V8-V9-V10-V11 (1$^{ère}$ série, connexions de 1 en 1)

V1-V3-V5-V7-V9-V11-V2-V4-V6-V8-V10 (2$^{ème}$ série, connexions de 2 en 2)

V1-V4-V7-V10-V2-V5-V8-V11-V3-V6-V9 (3$^{ème}$ série, connexions de 3 en 3)

V1-V5-V9-V2-V6-V10-V3-V7-V11-V4-V8 (4$^{ème}$ série, connexions de 4 en 4)

V1-V6-V11-V5-V10-V4-V9-V3-V8-V2-V7 (5$^{ème}$ série, connexions de 5 en 5)

Là encore, on peut vérifier que chaque doublet i-j apparait une fois et une seule dans la liste.

Cependant, si tous les couples/doublets possibles tels que visés ci-dessus sont utilisés et si deux particules traversent un dispositif de détection équipé d'un tel circuit de connexion simultanément, une ambiguïté de localisation peut éventuellement apparaître, dans ce cas il ne devient plus possible de localiser avec précision les particules. Par exemple, si deux particules sont réellement reçues au niveau de zones Z1 et Z2 (figure 3), elles laissent respectivement un signal sur les voies V1, V2, V3, et V5, trois solutions de localisation sont alors possibles : (V1 ; V2) ; (V3 ; V5) ou (V1 ; V5) ;(V3 ; V2) ou (V1 ; V3) ;(V2 ; V5) correspondant respectivement aux zones Z1 et Z2, ou aux zones Z3, Z4, ou aux zones Z5 et Z6. Dans ce cas particulier, il n'est pas possible de dire quelle solution est la bonne, puisque tous ces groupes de voies de sortie apparaissent dans le dispositif de détection. En revanche, il est facile de retirer une partie des groupes de voies de sortie lors de la fabrication du circuit, notamment des connexions du circuit, ce qui permet de lever des ambiguïtés quand plusieurs particules traversent le détecteur simultanément. Autrement dit, dans le cadre d'un dispositif de détection de particule(s) (permettant notamment de localiser une ou plusieurs particules) équipé d'un tel circuit de connexion, les points de connexion 103 du circuit de connexion sont tels que seule une fraction des groupes de voies de sortie est formée de sorte que lorsque deux particules sont reçues dans n'importe quelle zone d'un élément de détection associé à ladite pluralité de canaux, elles sont localisées en diminuant les possibilités d'ambigüité. Bien entendu, ce retrait de certains des doublets se fait au détriment du nombre de voies de sortie.

En revenant à un cas un peu plus général et en considérant que le nombre de voies de sortie de chaque groupe de voies de sortie est égal au nombre de canaux de chaque groupe de canaux, le circuit peut comporter z groupes de voies de sortie différents non ordonnés avec z un nombre entier quelconque choisi indifféremment entre 2 et $C_n^k$ (autrement dit $2 \leq z \leq C_n^k$ ).

De préférence, p est égal à z+k-1. Ce cas particulier permet d'économiser le plus de voies de sortie.

Selon une mise œuvre, deux canaux appartenant à deux groupes de canaux différents et reliés à une même voie de sortie sont séparés d'au moins x canaux distinct, notamment avec x supérieur à un nombre maximal de canaux ayant détecté une particule dans le cadre d'un dispositif de détection/localisation de particule(s) (autrement dit, x est supérieur au nombre de canaux de chaque groupe de canaux). Ceci permet d'éloigner au maximum deux canaux connectés à la même voie de sortie, notamment afin d'éviter une localisation ambiguë lorsque le circuit de connexion est utilisé dans le cadre d'un dispositif de détection de particule(s).

De préférence, la pluralité de canaux est agencée de telle sorte que chacun des canaux de la pluralité de canaux est adjacent à au moins un autre canal de la pluralité de canaux et dans le cas des pistes (c'est-à-dire que les canaux sont en fait des pistes), au maximum adjacent à deux autres pistes de la pluralité de pistes. De préférence, les canaux sont sensiblement parallèles les uns aux autres. Autrement dit, les canaux de la pluralité de canaux peuvent être disposés côte à côte et échelonnés selon l'axe A1 des figures 3 et 4 (de préférence les canaux sont sensiblement perpendiculaires à l'axe A1). Les canaux peuvent être disposés sur une même face d'un même substrat de support 105. Le substrat de support peut être fonctionnalisé ou non. Par exemple, le substrat de support 105 est un circuit imprimé.

Un autre objet de l'invention est un dispositif de détection 106 (figure 3) d'au moins une particule. Un tel dispositif de détection comprend au moins un circuit de connexion 100 tel que décrit précédemment dont chacun des canaux 101 de la pluralité de canaux forme un canal de détection de particule (la particule pouvant être considérée comme détectée lorsqu'un signal se propage dans un ou plusieurs canaux). Autrement dit, une particule peut être considérée comme détectée lorsqu'elle est reçue par ledit dispositif au niveau du canal de détection considéré.

En fait, par canaux de détection de particule(s) ou configurés de sorte à détecter au moins une particule, on entend un canal apte à récupérer un signal par exemple généré dans un milieu de détection lorsque ladite particule atteint le milieu de détection. Par exemple, si la particule interagit dans le milieu de détection, ce dernier peut générer, à partir de la particule, soit des charges soit des photons qui sont récupérées par le ou les canaux afin de constituer un signal d'intérêt à analyser pour localiser la particule ainsi détectée. Cette récupération se fait généralement uniquement au niveau de l'endroit où la particule a interagit.

En outre, le dispositif de détection comprend aussi avantageusement un élément de détection 107 destiné à recevoir ladite au moins une particule à localiser et cet élément de détection comporte la pluralité de canaux de détection de sorte qu'une particule est détectée par la propagation d'un signal de détection dans au moins deux canaux de détection adjacents. L'élément de détection 107 peut alors comporter le substrat de support 105 tel que défini ci-dessus de sorte que les canaux de détection s'étendent sur une même face 107a dudit substrat de support 105 et soient associés à un milieu de détection recouvrant de préférence les canaux 101.

Par ailleurs, le dispositif de détection comporte un composant d'analyse 108 relié à chacune des voies de sortie de la pluralité de voies de sortie. Ce composant d'analyse 108 permet de localiser une particule lorsque cette dernière est détectée par l'élément de détection 107.

De préférence, le dispositif de détection 106 est configuré de sorte que ladite particule à localiser est détectée par au moins tous les canaux d'un des groupes de canaux de détection adjacents (notamment par propagation d'un signal de détection dans chacun des canaux dudit groupe de canaux). Cette configuration est rendue possible en calibrant le pas de séparation des canaux adjacents et/ou les dimensions des canaux adjacents en fonction de l'extension spatiale du signal généré par la ou les particules que l'on cherche à détecter pour que lorsqu'une particule est reçue par l'élément de détection, le signal impacte au moins tous les canaux d'un même groupe de canaux. Ainsi, il est donc intéressant d'étaler le signal afin de toucher au moins deux canaux. Selon une variante, il est aussi possible de recouvrir les canaux par un élément d'étalement du signal permettant d'augmenter le nombre de canaux recevant le signal, par exemple en utilisant un élément d'étalement sous forme d'une pâte résistive ou d'un film résistif dans le cas d'un circuit de connexion électrique.

Par « particule reçue », on entend une particule qui atteint l'élément de détection 107 associé aux canaux de détection 101 de la pluralité de canaux. La particule peut traverser ou non l'élément de détection 107.

Dans le cas où les particules n'arrivent pas simultanément, le dispositif de détection est aussi configuré de sorte que la particule détectée est localisée, par le composant d'analyse 108, dans une unique zone de l'élément de détection 107 uniquement à partir de données de détection issues des canaux de détection 101 ayant détecté (par propagation du signal de détection) ladite particule. Ces données de détection sont transmises audit composant d'analyse 108 par les voies de sortie 102 du groupe de voies de sortie associé au groupe de canaux ayant détecté ladite particule. En fait, selon une réalisation particulière, le composant d'analyse 108 comprend un élément de localisation associant à chaque particule détectée une unique zone de l'élément de détection 107, cette association étant déterminée à partir des données de détection transmises par les voies de sortie du groupe de voie de sortie associé au groupe de canaux ayant détecté ladite particule.

Par « unique zone », on entend un unique ensemble (ou unique groupe) de canaux adjacents de l'élément de détection 107. Cet élément de détection 107 peut alors être divisé en plusieurs zones distinctes, et lorsqu'une particule est reçue par l'élément de détection on va chercher par la suite à la localiser pour savoir dans quelle zone elle a été effectivement reçue.

De manière plus générale et en faisant abstraction du circuit de connexion décrit ci-dessus, le dispositif de détection peut comporter un élément de détection destiné à recevoir la particule à localiser. L'élément de détection comporte aussi une pluralité de canaux de détection par exemple disposés sur une même face de l'élément de détection. Le dispositif de détection comporte une pluralité de voies de sortie dont au moins une est connectée à au moins deux canaux de la pluralité de canaux. Chacun des canaux de la pluralité de canaux est connecté à une unique voie de sortie de la pluralité de voies de sortie. Par ailleurs, le dispositif de détection comprend un composant d'analyse relié à chacune des voies de sortie de la pluralité de voies de sortie. Les connexions entre les canaux et les voies de sorties sont telles que le composant d'analyse est configuré de sorte à déterminer une zone de localisation unique de la particule reçue par l'élément de détection uniquement à partir de données générées par lesdits canaux de détection de la pluralité de canaux de détection et transmises audit composant d'analyse par les voies de sorties associées aux canaux de détection ayant détecté ladite au moins une particule.

En fait, on comprend que, contrairement à l'art antérieur, il est possible de détecter et de localiser une particule dans une unique zone de l'élément de détection sans avoir besoin d'équiper les deux faces opposées de l'élément de détection avec des canaux de détection.

Comme évoqué précédemment, même s'il est possible de réaliser jusqu'à $C_n^k = \dfrac{n!}{k!(n-k)!}$ combinaisons possibles de groupes de voies de sortie différents et non ordonnés, il est possible d'ajuster le nombre de groupes réellement utilisés dans le dispositif de détection 106 afin de lever des ambiguïtés lorsque plusieurs particules sont reçues par l'élément de détection simultanément. Dans le cas particulier de triplets (groupes de voies de trois voies et groupes de canaux de trois canaux), il n'est pas possible de construire une liste contenant tous les triplets possibles, mais des constructions similaires à celles présentées précédemment arrivent à former une liste de connexions contenant plus de 75% des triplets possibles. Ainsi, avec n=64, on peut construire un dispositif de détection 106 de plus de 32000 canaux de détection, dans lequel n'importe quel triplet de voies n'est présent au plus qu'une fois dans ledit dispositif de détection 106.

Un procédé de localisation d'au moins une particule peut être réalisé à partir d'un dispositif de détection 106 tel que décrit ci-dessus dans ses différentes réalisations. Un tel procédé peut comprendre : une étape dans laquelle ladite au moins une particule est reçue par l'élément de détection 107 ; une étape de détection par au moins tous les canaux 101 de détection d'un même groupe de canaux adjacents de ladite au moins une particule (que cela soit par détection directe ou via l'élément d'étalement visé précédemment lors de la propagation du signal de détection à partir d'informations issues du milieu de détection) ; une étape de génération de données de détection par chacun des canaux de détection 101 du groupe de canaux de détection adjacents ayant détecté ladite au moins une particule ; une étape de réception par le composant d'analyse 108 desdites données de détection transmises par les voies de sortie 102 connectées audit groupe de canaux de détection adjacents ayant détecté ladite au moins une particule ; une étape de détermination, à partir desdites données de détection reçues, d'un groupe de voies de sortie associé ; une étape de détermination d'une zone de l'élément de détection comprenant le groupe de canaux de détection adjacents connecté au groupe de voies de sortie déterminé, ladite zone de l'élément de détection fournissant une donnée de localisation de ladite au moins une particule. La zone de l'élément de détection fournissant une donnée de localisation de ladite au moins une particule correspond en fait à « l'unique zone » décrite ci-avant. De préférence, la zone déterminée dans laquelle est localisée la particule peut être issue d'une cartographie préétablie associant à chaque groupe de voies de sortie une unique zone de localisation.

Un support d'enregistrement de données lisible par un calculateur, sur lequel est enregistré un programme informatique peut comprendre des moyens de codes de programme informatique de mise en œuvre des étapes du procédé tel que décrit.

Un programme informatique peut comprendre un moyen de codes de programme informatique adapté à la réalisation des étapes du procédé tel que décrit lorsque le programme est exécuté par un calculateur.

La plupart des, voire toutes les, expériences de physique de particules pourraient mettre en œuvre ce genre de circuit de connexion en vue de détecter lesdites particules, à des degrés divers selon le flux de particules. Il existe toujours des zones où le flux de particules est moins élevé, et où ce principe pourrait être appliqué avec succès. L'imagerie pourrait aussi mettre à profit le multiplexage dudit circuit de connexion, notamment en tomographie muonique ou en imagerie médicale. De nouvelles expériences deviennent également réalisables, par exemple la construction d'ensemble de détection de très grande surface pour l'étude des gerbes du rayonnement cosmique. Enfin, le remplacement de détecteurs de type chambre à fils par des dispositifs de détection à micro-pistes (remplacement souvent prohibitif à cause de la multiplication du nombre de voies) devient envisageable, avec à la clé une amélioration sensible de la résolution en position.

Dans la présente description, un dispositif de détection permet notamment de localiser une ou plusieurs particules.

## Revendications

1. Circuit de connexion (100) multiplexé comprenant une pluralité de canaux munie de p canaux (101), notamment configurés de sorte à détecter au moins une particule, et une pluralité de voies de sortie munie de n voies de sortie (102), avec p>n, chaque canal étant connecté à une unique voie de sortie (102), **caractérisé en ce qu'**il comprend des groupes de canaux adjacents, chaque canal (101) du circuit de connexion (100) appartenant à au moins un groupe de canaux adjacents et chaque groupe de canaux adjacents étant défini par un ensemble formé par les connexions des canaux (101) du groupe de canaux adjacents aux voies de sortie (102) correspondantes, cet ensemble formant un groupe de voies de sortie associé au groupe de canaux adjacents, et **en ce que** chaque groupe de canaux adjacents est associé à un groupe de voies de sortie unique, différent de celui des autres groupes de canaux adjacents.

2. Circuit de connexion (100) selon la revendication 1, **caractérisé en ce que** le pas de séparation et/ou les dimensions des canaux adjacents est fonction de l'extension spatiale d'un signal généré par la au moins une particule à détecter de sorte qu'un tel signal impacte au moins tous les canaux d'un même groupe de canaux adjacents.

3. Circuit de connexion (100) selon la revendication 1 ou 2, **caractérisé en ce qu'**il comporte des points de connexion (103) entre les canaux (101) et les voies de sortie (102) permettant de réaliser la connexion de chaque canal de la pluralité de canaux à une unique voie de sortie (102) de la pluralité de voies de sortie, et **en ce que** les points de connexion (103) :

   - définissent les groupes de canaux adjacents,
   - forment les groupes de voies de sortie, lesdites voies de sortie de chacun des groupes de voies de sortie étant choisies parmi les n voies de sortie (102) de la pluralité de voies de sortie sans que l'ordre de la voie de sortie dans le groupe de voies de sortie n'ait d'importance et de sorte que les groupes de voies de sortie sont tous différents les uns des autres,

un groupe de voies de sortie et un groupe de canaux associé comprenant respectivement un nombre de voies de sortie (102) et un nombre de canaux (101) égaux.

4. Circuit selon l'une des revendications précédentes, **caractérisé en ce que** au moins une des voies de sortie (102) de la pluralité de voies de sortie est connectée à au moins deux canaux (101) de la pluralité de canaux de deux groupes de canaux distincts.

5. Circuit selon l'une des revendications précédentes, **caractérisé en ce que** chaque groupe de canaux comprend k canaux et **en ce que** chaque groupe de voies de sortie comporte k voies de sortie avec k≥2.

6. Circuit selon la revendication précédente, **caractérisé en ce que** k étant égal à 2, les connexions (103) sont réalisées de sorte que la pluralité de canaux comporte indifféremment entre 2 et

$$\frac{n*(n-1)}{2}+1 \text{ canaux.}$$

7. Circuit selon la revendication 5, **caractérisé en ce qu'**il comporte z groupes de voies de sortie différents non ordonnés avec z un nombre entier quelconque choisi indifféremment entre 2 et $C_n^k$.

8. Circuit selon la revendication précédente, **caractérisé en ce que** p=z+k-1.

9. Circuit selon l'une quelconque des revendications précédentes, **caractérisé en ce que** deux canaux appartenant à deux groupes de canaux différents et reliés à une même voie de sortie sont séparés d'au moins x canaux distincts, notamment avec x supérieur au nombre de canaux de chaque groupe de canaux.

10. Circuit selon l'une quelconque des revendications précédentes, **caractérisé en ce que** la pluralité de canaux est agencée de telle sorte que chacun des canaux de la pluralité de canaux est adjacent à au moins un, et de préférence sensiblement parallèle, autre canal de la pluralité de canaux.

11. Circuit selon la revendication précédente, **caractérisé en ce que** les canaux sont des pistes, et **en ce que** chacune des pistes est au maximum adjacente à deux autres pistes.

12. Dispositif de détection d'au moins une particule **caractérisé en ce qu'**il comprend au moins un circuit de connexion (100) selon l'une quelconque des revendications précédentes dont chacun des canaux (101) de la pluralité de canaux forme un canal de détection de particule, et **en ce qu'**il comprend :

- un élément de détection (107) destiné à recevoir ladite au moins une particule à localiser et comportant la pluralité de canaux de détection de sorte qu'une particule est détectée par la propagation d'un signal de détection dans au moins deux canaux de détection adjacents et,
- un composant d'analyse (108) relié à chacune des voies de sortie de la pluralité de voies de sortie.

13. Dispositif selon la revendication précédente, **caractérisé en ce qu'**il est configuré de sorte que ladite particule à localiser est détectée par au moins tous les canaux (101) d'un des groupes de canaux de détection adjacents, et que la particule détectée est localisée, par le composant d'analyse (108), dans une unique zone de l'élément de détection (107) uniquement à partir de données de détection issues des canaux de détection (101) ayant détecté ladite particule et transmises audit composant d'analyse (108) par les voies de sortie (102) du groupe de voies de sortie associé au groupe de canaux ayant détecté ladite particule.

14. Procédé de localisation d'au moins une particule à partir d'un dispositif selon l'une quelconque des revendications 12 à 13, **caractérisé en ce qu'**il comporte :

- une étape dans laquelle ladite au moins une particule est reçue par l'élément de détection (107),
- une étape de détection par au moins tous les canaux (101) de détection d'un même groupe de canaux adjacents de ladite au moins une particule,
- une étape de génération de données de détection par chacun des canaux (101) de détection du groupe de canaux de détection adjacents ayant détecté ladite au moins une particule,
- une étape de réception par le composant d'analyse (108) desdites données de détection transmises par les voies de sortie (102) connectées audit groupe de canaux de détection adjacents ayant détecté ladite au moins une particule,
- une étape de détermination, à partir desdites données de détection reçues, d'un groupe de voies de sortie associé,
- une étape de détermination d'une zone de l'élément de détection comprenant le groupe de canaux de détection adjacents connecté au groupe de voies de sortie déterminé, ladite zone de l'élément de détection fournissant une donnée de localisation de ladite au moins une particule.

**Patentansprüche**

1. Multiplex-Verbindungsschaltung (100), die eine Vielzahl von Kanälen aufweist, die mit p Kanälen (101) ausgestattet ist, insbesondere ausgebildet zum Erkennen mindestens eines Partikels, und eine Vielzahl von Ausgangswegen, die mit n Ausgangswegen (102) ausgestattet ist, wobei p>n und jeder Kanal mit einem einzigen Ausgangsweg (102) verbunden ist, **dadurch gekennzeichnet, dass** sie Gruppen von angrenzenden Kanälen aufweist, wobei jeder Kanal (101) der Verbindungsschaltung (100) zu mindestens einer Gruppe von angrenzenden Kanälen gehört und jede Gruppe von angrenzenden Kanälen durch eine Menge definiert wird, die durch die Verbindungen der Kanäle (101) der Gruppe von angrenzenden Kanälen zu den entsprechenden Ausgangswegen (102) gebildet wird, wobei diese Menge eine Gruppe von Ausgangswegen bildet, die der Gruppe von angrenzenden Kanälen zugeordnet ist, und dass jede Gruppe von angrenzenden Kanälen einer einzelnen Gruppe von Ausgangswegen zugeordnet ist, die sich von der der anderen Gruppen von angrenzenden Kanälen unterscheidet.

2. Verbindungsschaltung (100) nach Anspruch 1, **dadurch gekennzeichnet, dass** der Trennabstand und/oder die Abmessungen der angrenzenden Kanäle eine Funktion der räumlichen Ausdehnung eines Signals sind, das durch das mindestens eine zu erkennende Partikel in der Art erzeugt wird, dass ein solches Signal mindestens alle Kanäle derselben Gruppe von angrenzenden Kanälen beeinflusst.

3. Verbindungsschaltung (100) nach Anspruch 1 oder 2, **dadurch gekennzeichnet, dass** sie Verbindungspunkte (103) zwischen den Kanälen (101) und den Ausgangswegen (102) aufweist, die es ermöglichen, die Verbindung zwischen jedem Kanal der Vielzahl von Kanälen zu einem einzigen Ausgangsweg (102) der Vielzahl von Ausgangswegen durchzuführen, und dass die Verbindungspunkte (103):

    - die Gruppen von angrenzenden Kanälen definieren,
    - die Gruppen von Ausgangswegen bilden, wobei die Ausgangswege jeder der Gruppen von Ausgangswegen aus den n Ausgangswegen (102) der Vielzahl von Ausgangswegen ausgewählt werden, ohne dass die Reihenfolge des Ausgangswegs in der Gruppe von Ausgangswegen von Bedeutung ist und in der Art, dass sich die Gruppen von Ausgangswegen alle voneinander unterscheiden,

    wobei eine Gruppe von Ausgangswegen und eine zugeordnete Gruppe von Kanälen jeweils eine Anzahl von Ausgangswegen (102) und eine Anzahl von Kanälen (101) aufweist, die gleich sind.

4. Schaltung nach einem der vorausgehenden Ansprüche, **dadurch gekennzeichnet, dass** mindestens einer der Ausgangswege (102) der Vielzahl von Ausgangswegen mit mindestens zwei Kanälen (101) der Vielzahl von Kanälen von zwei verschiedenen Gruppen von Kanälen verbunden ist.

5. Schaltung nach einem der vorausgehenden Ansprüche, **dadurch gekennzeichnet, dass** jede Gruppe von Kanälen k Kanäle aufweist und dass jede Gruppe von Ausgangswegen k Ausgangswege mit $k \geq 2$ aufweist.

6. Schaltung nach dem vorausgehenden Anspruch, **dadurch gekennzeichnet, dass** k gleich 2 ist und die Verbindungen (103) in der Art ausgeführt sind, dass die Vielzahl von Kanälen gleichermaßen zwischen 2 und $\dfrac{n*(n-1)}{2}+1$ Kanäle aufweist.

7. Schaltung nach Anspruch 5, **dadurch gekennzeichnet, dass** sie z Gruppen von unterschiedlichen nicht geordneten Ausgangswegen aufweist, wobei z eine beliebige ganze Zahl ist, die gleichermaßen zwischen 2 und $C_n^k$ gewählt wird.

8. Schaltung nach dem vorausgehenden Anspruch, **dadurch gekennzeichnet, dass** p = z + k - 1.

9. Schaltung nach einem der vorausgehenden Ansprüche, **dadurch gekennzeichnet, dass** zwei Kanäle, die zu zwei unterschiedlichen Gruppen von Kanälen gehören und mit demselben Ausgangsweg verbunden sind, durch mindestens x verschiedene Kanäle getrennt sind, wobei insbesondere x größer ist als die Anzahl der Kanäle von jeder Gruppe von Kanälen.

10. Schaltung nach einem der vorausgehenden Ansprüche, **dadurch gekennzeichnet, dass** die Vielzahl von Kanälen derart angeordnet ist, dass jeder der Kanäle der Vielzahl von Kanälen an mindestens einen und vorzugsweise im Wesentlichen parallelen anderen Kanal der Vielzahl von Kanälen angrenzt.

11. Schaltung nach dem vorausgehenden Anspruch, **dadurch gekennzeichnet,** die Kanäle Spuren sind und dass jede der Spuren höchstens an zwei andere Spuren angrenzt.

12. Vorrichtung zum Erkennen von mindestens einem Partikel, **dadurch gekennzeichnet, dass** sie mindestens eine Verbindungsschaltung (100) nach einem der vorausgehenden Ansprüche aufweist, wobei jeder der Kanäle (101) der Vielzahl von Kanälen einen Kanal zum Erkennen eines Partikels bildet, und dass sie Folgendes aufweist:

- ein Element zum Erkennen (107), bestimmt zum Empfangen des mindestens einen zu lokalisierenden Partikels, das die Vielzahl der Kanäle zum Erkennen in der Art aufweist, dass ein Partikel durch die Ausbreitung eines Signals zum Erkennen in mindestens zwei angrenzenden Kanälen zum Erkennen erkannt wird, und,
- eine Analysekomponente (108), die mit jedem der Ausgangswege der Vielzahl von Ausgangswegen verbunden ist.

13. Vorrichtung nach dem vorausgehenden Anspruch, **dadurch gekennzeichnet, dass** sie ausgebildet ist, dass das zu lokalisierende Partikel von mindestens allen Kanälen (101) einer der Gruppen von angrenzenden Kanälen zum Erkennen erkannt wird, und dass das erkannte Partikel von der Analysekomponente (108) in einer einzigen Zone des Elements zum Erkennen (107) ausschließlich ausgehend von den Daten zum Erkennen lokalisiert wird, die von den Kanälen zum Erkennen (101) ausgegeben werden, die das Partikel erkannt haben, und an die Analysekomponente (108) über die Ausgangswege (102) der Gruppe von Ausgangswegen übertragen werden, die der Gruppe von Kanälen zugeordnet ist, die das Partikel erkannt haben.

14. Verfahren zum Lokalisieren von mindestens einem Partikel ausgehend von einer Vorrichtung nach einem der Ansprüche 12 bis 13, **dadurch gekennzeichnet, dass** es Folgendes aufweist:

- einen Schritt, in dem das mindestens eine Partikel von dem Element zum Erkennen (107) erhalten wird,
- einen Schritt zum Erkennen des mindestens einen Partikels durch mindestens alle Kanäle (101) zum Erkennen derselben Gruppe von angrenzenden Kanälen,
- einen Schritt zum Erzeugen der Daten zum Erkennen durch jeden der Kanäle zum Erkennen (101) der Gruppe von angrenzenden Kanälen zum Erkennen, die das mindestens eine Partikel erkannt haben,
- einen Schritt zum Empfangen durch die Analysekomponente (108) der Daten zum Erkennen, die von den Ausgangswegen (102) übertragen werden, die mit der Gruppe von angrenzenden Kanälen zum Erkennen verbunden sind, die das mindestens eine Partikel erkannt haben,
- einen Schritt zum Bestimmen einer Gruppe von zugeordneten Ausgangswegen ausgehend von den empfangenen Daten zum Erkennen, wobei ein Schritt zum Bestimmen einer Zone des Elements zum Bestimmen die Gruppe von angrenzenden Kanälen zum Erkennen aufweist, die mit der bestimmten Gruppe von Ausgangswegen verbunden ist, wobei die Zone des Elements zum Bestimmen ein Datenelement zum Lokalisieren des mindestens einen Partikels liefert.

**Claims**

1. Multiplexed connection circuit (100) comprising a plurality of channels furnished with p channels (101), in particular configured so as to detect at least one particle, and a plurality of output pathways furnished with n output pathways (102), with p>n, each channel being connected to a lone output pathway (102), **characterized in that** it comprises groups of adjacent channels, each channel (101) of the connection circuit (100) belonging to at least one group of adjacent channels and each group of adjacent channels being defined by an assembly formed by the connections of the channels (101) of the group of adjacent channels to the corresponding output pathways (102), this assembly forming a group of output pathways which is associated with the group of adjacent channels, and **in that** each group of adjacent channels is associated with a single group of output pathways, different from that of the other groups of adjacent channels.

2. Connection circuit (100) according to Claim 1, **characterized in that** the spacing separating the adjacent channels and/or the dimensions of the adjacent channels is a function of the spatial extent of the signal generated by the at least one particle that it is sought to detect so that such signal impacts at least all the channels of one and the same group of adjacent channels.

3. Connection circuit (100) according to Claim 1 or 2, **characterized in that** it comprises connection points (103) between the channels (101) and the output pathways (102) making it possible to carry out the connection of each channel of the plurality of channels to a lone output pathway (102) of the plurality of output pathways, and **in that** the connection points (103):

- define the groups of adjacent channels,
- form the groups of output pathways, the said output pathways of each of the groups of output pathways being chosen from among the n output pathways (102) of the plurality of output pathways without the order of the output pathway in the group of output pathways having any importance and so that the groups of output pathways are all different from one another,

a group of output pathways and an associated group of channels comprising respectively a number of output pathways (102) and a number of channels (101) which are equal.

4. Circuit according to one of the preceding claims, **characterized in that** at least one of the output pathways (102) of the plurality of output pathways is connected to at least two channels (101) of the plurality of channels of two groups of distinct channels.

5. Circuit according to one of the preceding claims, **characterized in that** each group of channels comprises k channels and **in that** each group of output pathways comprises k output pathways with $k \geq 2$.

6. Circuit according to the preceding claim, **characterized in that** k being equal to 2, the connections (103) are carried out so that the plurality of channels comprises immaterially between 2 and $\frac{n*(n-1)}{2}+1$ channels.

7. Circuit according to Claim 5, **characterized in that** it comprises z unordered different groups of output pathways with z any integer number chosen immaterially between 2 and $C_n^k$ .

8. Circuit according to the preceding claim, **characterized in that** p=z+k-1.

9. Circuit according to any one of the preceding claims, **characterized in that** two channels belonging to two groups of different channels and linked to one and the same output pathway are separated by at least x distinct channels, in particular with x greater than the number of channels of each group of channels.

10. Circuit according to any one of the preceding claims, **characterized in that** the plurality of channels is arranged in such a way that each of the channels of the plurality of channels is adjacent to at least one, and preferably substantially parallel, other channel of the plurality of channels.

11. Circuit according to the preceding claim, **characterized in that** the channels are tracks, and **in that** each of the tracks is at the maximum adjacent to two other tracks.

12. Device for detecting at least one particle, **characterized in that** it comprises at least one connection circuit (100) according to any one of the preceding claims of which each of the channels (101) of the plurality of channels forms a particle detection channel, and **in that** it comprises:

- a detection element (107) intended to receive the said at least one particle to be located and comprising the plurality of detection channels so that a particle is detected by the propagation of a detection signal in at least two adjacent detection channels, and
- an analysis component (108) linked to each of the output pathways of the plurality of output pathways.

13. Device according to the preceding claim, **characterized in that** it is configured so that the said particle to be located is detected by at least all the channels (101) of one of the groups of adjacent detection channels, and that the detected particle is located, by the analysis component (108), in a lone zone of the detection element (107) solely on the basis of detection data arising from the detection channels (101) having detected the said particle and transmitted to the said analysis component (108) by the output pathways (102) of the group of output pathways which is associated with the group of channels that has detected the said particle.

14. Method for locating at least one particle on the basis of a device according to any one of Claims 12 to 13, **characterized in that** it comprises:

- a step in which the said at least one particle is received by the detection element (107),
- a step of detection by at least all the detection channels (101) of one and the same group of adjacent channels of the said at least one particle,
- a step of generating detection data by each of the detection channels (101) of the group of adjacent detection channels that has detected the said at least one particle,
- a step of reception by the analysis component (108) of the said detection data transmitted by the output pathways (102) connected to the said group of adjacent detection channels that has detected the said at least one particle,
- a step of determination, on the basis of the said detection data received, of an associated group of output pathways,
- a step of determining a zone of the detection element comprising the group of adjacent detection channels which is connected to the determined group of output pathways, the said zone of the detection element providing an item of data regarding location of the said at least one particle.

FIG.1 (Art antérieur)

FIG.2 (Art antérieur)

FIG.3

FIG.4

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- DE 102005055656 B3